(19) **Europäisches Patentamt European Patent Office Office européen des brevets**

(11) **EP 4 027 877 B1**

(12) **EUROPEAN PATENT SPECIFICATION**

(45) Date of publication and mention
of the grant of the patent:
**23.04.2025 Bulletin 2025/17**

(21) Application number: **20772375.0**

(22) Date of filing: **09.09.2020**

(51) International Patent Classification (IPC):
**A61B 5/107** (2006.01)   **A61F 5/01** (2006.01)
**A61B 5/00** (2006.01)   **A61B 5/01** (2006.01)
**A61B 5/11** (2006.01)

(52) Cooperative Patent Classification (CPC):
**A61B 5/107; A61B 5/01; A61B 5/6804;**
**A61F 5/0102;** A61B 5/0022; A61B 5/1073;
A61B 5/1114; A61B 2562/0247; A61B 2562/0261;
A61B 2562/0271; G16H 50/50

(86) International application number:
**PCT/GB2020/052160**

(87) International publication number:
**WO 2021/048538 (18.03.2021 Gazette 2021/11)**

(54) **FABRICATION OF CUSTOM ORTHOSIS**

HERSTELLUNG VON KUNDENSPEZIFISCHEN ORTHESEN

FABRICATION D'UNE ORTHÈSE PERSONNALISÉE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.09.2019 GB 201913032**

(43) Date of publication of application:
**20.07.2022 Bulletin 2022/29**

(73) Proprietors:
• **John Florence Limited
Haywards Heath
West Sussex RH16 1PH (GB)**
• **The University of Sussex
Brighton BN1 9RH (GB)**

(72) Inventors:
• **PORTER, Stephen
Sussex RH16 1PH (GB)**
• **MUNZENRIEDER, Niko
Sussex BN1 9QF (GB)**
• **ROGGEN, Daniel
Sussex BN1 9QF (GB)**
• **LUGODA, Don Pasindu Vijai
Sussex BN1 9QF (GB)**
• **GARCIA-GARCIA, Leonardo
Sussex BN1 9QF (GB)**
• **COSTA, Julio
Sussex BN1 9QF (GB)**

(74) Representative: **Richards, Michèle E. et al
Fry Heath & Spence LLP
Unit A, Faraday Court
Faraday Road
Crawley, West Sussex RH10 9PU (GB)**

(56) References cited:
WO-A1-2009/023937     WO-A2-2012/168836
US-A1- 2014 276 235     US-A1- 2019 046 114
US-B2- 7 145 432

- **LUGODA PASINDU ET AL: "ShapeSense3D textile-sensing and reconstruction of body geometries", UBICOMP/ISWC '19 ADJUNCT: ADJUNCT PROCEEDINGS OF THE 2019 ACM INTERNATIONAL JOINT CONFERENCE ON PERVASIVE AND UBIQUITOUS COMPUTING AND PROCEEDINGS OF THE 2019 ACM INTERNATIONAL SYMPOSIUM ON WEARABLE COMPUTERS, 9 September 2019 (2019-09-09), pages 133 - 136, XP058472191, ISBN: 978-1-4503-6869-8, DOI: 10.1145/ 3341162.3343846**

**Description**

BACKGROUND

Orthotic products (orthoses) may be used to support, brace and/or improve functions of parts of the body. Exemplary orthoses include upper-limb orthoses, lower-limb orthoses, spinal and neck orthoses, wrist orthoses, ankle orthoses and similar applications.

The traditional method for the manufacture of orthoses is plaster casting but this method is time and resource consuming. The plaster cast method has been followed by use of optical body scanning for digital anatomical image capture.

A target body surface will typically be scanned directly or indirectly (from a mould) to generate a three-dimensional data set representing the body surface geometry. Three-dimensional design software (CAD) may then be used to design the body scaffold and three-dimensional printing may be used for fabricating personalized orthoses custom built for individual patient anatomies.

Direct body scanning has inherent inefficiencies and a limited use audience, however. This is particularly the case when optically scanning children with limited capacity for keeping still and those affected with neurological and neuromuscular medical conditions, such as cerebral palsy. In addition, a healthcare practitioner normally needs to apply force to support and/or correct anatomical body structures to define the shape of an orthosis. However, the healthcare practitioner is unable to directly apply corrective biomechanical forces to anatomical body structures and apply direct forces for correction of body parts as the healthcare practitioner's hands interfere and/or block the body-part scan. This issue prevents the efficient application of optical scanning because extensive post-processing is required to discriminate between the patient's body and the healthcare practitioner's hands. Furthermore, such imaging equipment is expensive and requires highly trained technicians.

An alternative to optical scanning is needed to achieve an easier and more efficient model of a body part in combination with a measurement of forces applied and used.

US2014/276235 and WO2012/168836 disclose a method for providing a customised orthotic product.

SUMMARY OF THE INVENTION

The invention is set out in the appended set of claims.

In particular, the invention is a method for providing a customised orthotic product, comprising providing an article, comprising a stretchable material, and a plurality of strain sensors positioned in contact with the material such that stretching of the material is detectable by the sensors; placing the article onto a body part so that the material is stretched, at least in part; providing a computing device operatively coupled to the article and configured to receive output data from the sensors and to process the data to determine the three-dimensional shape of the body part; applying force, deformation pressure, rotation and/or flexion at one or more target regions of the body part to hold, manipulate and/or correct the body part; reading output from the sensors, and determining the three-dimensional shape of the body part based on the output from the sensors; using the determined three-dimensional shape of the body part to design and/or fabricate a customized orthotic product.

In its broadest sense, one aspect of the disclosure is an article, comprising a stretchable material, and a plurality of sensors in contact with the material such that stretching of the material is detectable by the sensors. The sensors may detect stretching in one direction or in more than one direction in any two-dimensional plane.

The sensors may be integrated or embedded within the material and/or positioned onto the material surface. In one embodiment, the sensors are provided in channels, pockets, or the like, formed in the material. In another embodiment, the sensors are laminated onto or incorporated in an elastomeric material. For example, the sensors may be laminated onto an elastomeric sheet, where the sheet may be in the form of a strip. The elastomeric material may comprise silicone, polyurea, polyether, polyurethane, or the like, or any combination thereof.

In one embodiment, the material is a textile and the sensors are positioned on, or integrated or embedded within, the fibres or yarn of the textile. The stretchable material may comprise natural fibres, such as wool, cotton, etc. and/or synthetic fibres, such as elastane (a polyether-polyurea copolymer). By having the sensors positioned on or within the yarn, conventional textile manufacturing methods, like knitting and weaving, can be used to fabricate the article.

In one embodiment, the sensors are provided on or within a single or individual yarn (a "sensor yarn").

A flexible cover may be provided over the sensor yarn. Preferably, the flexible cover encapsulates the sensor yarn and, more preferably, provides a moisture/water-resistant/proof protective layer. In one embodiment, the flexible cover is a non-textile, for example, the flexible cover is a flexible adhesive, and preferably the flexible cover is itself covered with textile fibres.

In another embodiment, the sensors are coated or embedded in a water-proof material, for example, silicone. In this way, the article can be washed and re-used multiple times.

In one embodiment, the diameter of a covered sensor yarn in the textile material is about 2.0 mm or less, preferably about 0.7 mm to about 1.0 mm, such as about 0.75 mm to about 0.95 mm or about 0.8 mm to about 0.9 mm.

A string/plurality of sensors may be provided on a single yarn. Preferably, the sensors are positioned less than about 5 mm apart, such as about 4 mm, about 3 mm, about 2 mm or about 1 mm apart.

In one embodiment, the sensors have a height or thickness of less than about 0.02 mm, or less than about 0.015 mm or less than about 0.01 mm, such as 9 $\mu$m, 8 $\mu$m , 7 $\mu$m, 6 $\mu$m or 5 $\mu$m.

In one embodiment, the sensors have a width of less than about 1.5 mm, less than about 1.0 mm, less than about 0.85 mm, less than about 0.80 mm, or less than about 0.75 mm, such as 0.6 mm, 0.5 mm, 0.4 mm, 0.3 mm, 0.2 mm or 0.1 mm.

In one embodiment, the article is in the form of a sleeve adapted to snugly fit over a part of the object, for example a body part, such as a limb of a patient. In this embodiment, the plurality of sensors may comprise one or more closed loops of sensors.

In one embodiment, the article comprises a plurality of strain sensors to detect extension and at least one sensor selected from the group consisting of pressure/compression sensors, bend sensors, force sensors, temperature sensors. Preferably, the article comprises a combination of strain and pressure sensors. Preferably, the article comprises a plurality of strain sensors and at least one sensor configured to detect flexion, pressure, and rotation.

In one embodiment, the sensors are arranged in an array or matrix. Thus, rows and columns of sensors may be provided in or on the article material.

According to another aspect, the disclosure provides an apparatus for use in determining the three-dimensional shape of an object, which apparatus comprises:

> an article as described herein;
> a computing device operatively coupled to the article and configured to receive output data from the sensors and to process the data to determine the three-dimensional shape of the object.

The computing device preferably includes a display for displaying the three-dimensional image of the object. The computing device may be operatively coupled to a three-dimensional printer, a Computer Numerical Control (CNC) machine or a robot multi-axis carver, to provide a three-dimensional mould of the body part image and/or an orthotic product. The computing device preferably comprises a processor and storage. Stored instructions may be executed by the processor to perform an action. For example, stored instructions may be executed by the processor to perform an action on a three-dimensional printer responsive to input from a touch sensor.

In one embodiment, the object is a body part of a human or animal.

According to yet another aspect, the disclosure provides a computer-implemented method of generating a three-dimensional shape of an object using the apparatus as described hereinabove, which method comprises the steps of:

> placing the article onto the object so that the material is stretched, at least in part;
> reading output from the sensors; and determining the three-dimensional shape of the object based on the output from the sensors. The method may further comprise displaying the three-dimensional shape of the object on a display. The method may further comprise fabricating the object in three-dimensional form, for example by three-dimensional printing or by use of a CNC machine or a robot multi-axis carver.

According to another aspect, the disclosure provides a method for providing a customized orthotic product, especially splints and other body scaffolds, using the apparatus as described above, comprising:

> obtaining data representing the three-dimensional shape of a part of a body, optionally additionally obtaining data representing other information, such as at one or more target regions of the body part, from the sensors in the article; and
> using the three-dimensional shape of the body part, and optionally the other information, to produce the desired orthotic product.

In one embodiment, the three-dimension shape of the object is determined from sensor data using an algorithm or model that links inferred shape geometry to expected sensor recordings, and a loss function (L) and an optimisation function. The loss function (L) measures the degree of agreement between the sensor readings and the readings that would be expected based on the geometry of the inferred shape. The optimization function iteratively modifies the inferred shape in order to minimise the value of the loss function.

The methods according to the disclosure may comprise using the image of the three-dimensional shape of the body part to create a computer-aided image of the orthotic product and fabrication from the three-dimensional image by, for example, three-dimensional printing, or use of a CNC machine, or a robot multi-axis carver, to provide the orthotic device.

Alternatively, the method may comprise fabrication of the body part and using the physical three-dimensional body part to create the orthotic device.

According to the method, the data is output from a plurality of sensors provided in a stretchable material when the material is, in part or completely, stretched in position on a body part. In one embodiment, the data representing the three-dimensional shape of the object is obtained from output from strain and/or bend sensors. In one embodiment, the other information is data representing one or more target regions on the body part. The target regions are those regions of the body part where external force/pressure is applied to hold, manipulate and/or correct the body part. The material is placed on the body part so that adjacent the target regions there may be at least one sensor configured to detect pressure/compression, force, temperature, volume, flexion or rotation, provided in the stretchable material.

In one embodiment, the force at the target region is applied by a practitioner's hands. In one embodiment, the body part comprises one of an upper limb, a lower limb, a wrist, a neck, a splint and an ankle.

According to the disclosure, therefore, a selected body surface is effectively scanned from a wearable article (i.e., indirectly) to generate a three-dimensional data set representing the body surface geometry. The article containing embedded stretchable electronic sensors is applied to a three-dimensional object and sensor data is captured and relayed to a computer. Custom software and a specific processing algorithm reconstructs the data into an on-screen accurate digital geometric shape and replica three-dimensional image of the original three-dimensional object, with measured data information, to an on-screen monitor of a computer device. The article may have a connection for an integrated or external attached power supply. Wireless or wired technology may be used for data transfer from the article to a computer/ laptop/ smart device. For example, yarns may be connected to a Bluetooth™ - enabled interface hardware circuit that will condition the output signals and transmit them to smartphones or laptops for further processing.

The disclosure provides a series of different sized, selectable, textile stretchable and wearable garments containing a mesh of electronic stretchable and/or bendable sensors to apply over human/animal body parts/limbs or three-dimensional objects. The sensors from the garment relay information wirelessly to a computer device where custom computer software re-constructs accurate three-dimensional digital body geometric images, volumetric and strain force/pressure measurement data of the individual body-part/ limb or three-dimensional object being worn. The digital reconstructed geometric shaped and measured created data, images and/or files are used to assist healthcare practitioners provide biometric data for custom-made Computer Aided Design and Computer Aided Manufacture (CAD/CAM) and/or direct three-dimensional printing of custom-made/bespoke orthoses and prostheses products for the healthcare sector. The method and technology also provides healthcare practitioners and providers with detailed information that may enable individual custom selection of ready-made orthoses and prostheses products primarily for the healthcare sector.

According to a further aspect, the disclosure provides a method of monitoring or assessing applied skin strain, volume, pressure and/or force and other biomedical, biomechanical information, or motion-tracking, when an orthotic product is worn by a patient, the method comprising placing an article according to the invention as described herein on or over a body part, including on or over any identified target regions of the body part, under the orthotic product, and collecting data output from the sensors. The data is preferably collected over a period of days, weeks or months. The method enables real time on-screen motion tracking, monitoring, assessment and/or measurement. The data collected may be used to further enhance and influence, improve fitting and/or function of orthotic/prosthetic products and treatment.

The article according to the disclosure may further comprise sensors configured to detect skin temperature, skin surface sweat/perspiration, blood oxygenation, pH, chemicals, flexion, rotation, protonation and/or supination, providing data for further biomedical and clinical diagnostic, assessment, review, disease prevention and treatment purposes.

This disclosure provides a practical method, measurement tool and technology product for capturing biometric body-part data for the accurate geometric reconstruction of body-parts combined with other measurement information, including measurements of the deformation pressures being applied by the practitioner's hands for influencing, correcting, controlling, reshaping or supporting the patient limb/ body part.

The disclosure can also positively influence clinical assessment and provides an integrated, improved and more efficient method for capturing body part shape to improve treatment and care outcomes for individual users of orthoses and prostheses.

Definitions

Unless defined otherwise, all technical and scientific terms used herein have the same meaning as commonly understood by one of ordinary skill in the art.

Within this specification, the terms "comprises" and "comprising" are interpreted to mean "includes, among other things". These terms are not intended to be construed as "consists of only".

As used herein, the term "about" before a numeral is interpreted to mean plus (+) or minus (-) 10 to 50% of the numeral, for example + or - 20%, 30% or 40% of the numeral.

Within this specification embodiments have been described in a way which enables a clear and concise specification to be written. The following is a non-restrictive description of preferred embodiments of the invention, provided for the purpose of exemplification only, with reference to the accompanying drawings. It is intended and will be appreciated that embodiments may be variously combined or separated without parting from the invention.

BRIEF DESCRIPTION OF THE FIGURES

Figure 1 shows a sensing textile according to one embodiment of the disclosure and an enlarged view of a single yarn with sensors;

Figure 2 shows illustrations of the approach (as described below) applied to a 3D cone where (a) shows the result of physical simulation (target) and the reconstructed algorithm (inferred), (b) shows a sensing textile according to one embodiment of the disclosure as applied to a 3D printed version of that same cone, and (c) shows the result of the reconstructed algorithm applied on sensor measurements of (b);

Figures 3 (a) and (b) show an example of a resistance temperature detector strip for use in one embodiment of the disclosure, and (c) shows the strip embedded within a textile yarn; and

Figure 4 is a system architecture diagram showing examples of software and hardware that may be used in the apparatus and methods according to the disclosure.

DETAILED DESCRIPTION

The invention uses known techniques for fabricating personal three-dimensional printed orthotic products. The first step is to capture the anatomical geometry of the patient. In order to capture the personal three-dimensional geometry, an article according to the invention is placed onto or over the relevant body part in a stretched state. The second step is personal splint design; the physical structure is determined and modeled based on the three-dimensional geometry of the body part in combination with the additional information on target regions for a CAD designer to follow as instructions. The third step is three-dimensional printing of the personal orthotic. Any known three-dimensional printing technology can be used for the task. With further reference to the geometric data gathering stage, the goal is to capture a patient's desired (relevant) anatomic region with the use of the article according to the invention placed in contact with the region. The relevant anatomic region of the patient is usually stabilized when the article is in position and data from the stabilization as well as the shape of the region is captured. A practitioner positions the patient's anatomy and provides pressure or force at target regions. A CAD designer follows these instructions during the CAD design stage. Alternatively, the CAD software can be modified to take the output from the sensors, for example, the pressure (compression), tension, force or bend sensors, as commands with determined anatomic locations and generate the design.

Commercially available CAD software, such as Autodesk Fusion 360, Rhinoceros 5, and Solid Works, can be used to import the patient's three-dimensional body surface geometry and to design a suitable body scaffold.

A preliminary stretchable sleeve with a grid of strain sensors was tested on cylindrical objects and the method and results are described below. The sleeve and the reconstruction algorithm produced a cone with a given radius to an accuracy of 0.44 mm.

APPROACH

The shape reconstruction approach of the invention is based on the integration of a dense mesh/grid of sensors capable of measuring localised textile deformation in the textile sleeve. In one implementation such mesh is a matrix structure. Other implementations may include a honeycomb arrangement of sensors, or other shapes suitable to measure the textile deformation. Sensors may measure elongation, bending, shear, or others. In one implementation, the sensors are strain sensors which measures the local elongation of the textile as the difference between its rest and stretched state. Similarly, with other modalities, the sensors measure the difference between the current deformation and the rest state.

Hereafter, "inferred shape" refers to the shape of the sleeve which is reconstructed by a method which processes the sensor readings. This inferred shape is represented in the method of the invention by nodes, each of which has a coordinate in 3D space, the ensemble of which defines the inferred shape in 3D.

The number and arrangement of nodes relate to the number and arrangement of sensors integrated in the sleeve. Depending on the sensor modality, sensors may be located in between nodes (i.e. on the edge between nodes), or at the location of the nodes. In one implementation, strain sensors measure localised textile elongation, and are placed in between nodes.

The shape reconstruction method used in the invention consists of three elements:

1) A model which takes the geometry of an inferred shape, defined as the ensemble of 3D nodes coordinates, and which allows to obtain the readings of sensors which would be expected from the geometry of the inferred shape, according to their modality and arrangement;

2) A loss function L, which measures the degree of agreement between all the sensor readings, and the readings that would be expected based on the geometry of the inferred shape and obtained following (1);

3) An optimisation function, which will iteratively modify the position of the nodes of the inferred shape, in order to minimise the value of the loss function.

(1) is implemented by physical modelling principles or using lookup tables. For example, the distance between nodes can be linked to the elongation of strain sensors located in between two nodes using physical modelling. For other sensor modalities, the appropriate corresponding modelling is used. For (2), a general form of a loss function L is given below, which measures the agreement between N sensor readings, with $s_{inferred,i}$ the expected reading of sensor i, and $s_{target,i}$ the actual reading of sensor i. A loss function value of 0 indicates a perfect match between the expected reading of the sensors and the actual reading.

$$L = \sum_{i=1}^{N} (s_{inferred,i} - s_{target,i})^2$$

(3) is an optimisation algorithm, which iteratively modifies the 3D coordinates of the nodes such that the function L is minimised. This optimisation algorithm can be a gradient descent algorithm, a population-based optimisation algorithm such as a genetic algorithm, or others.

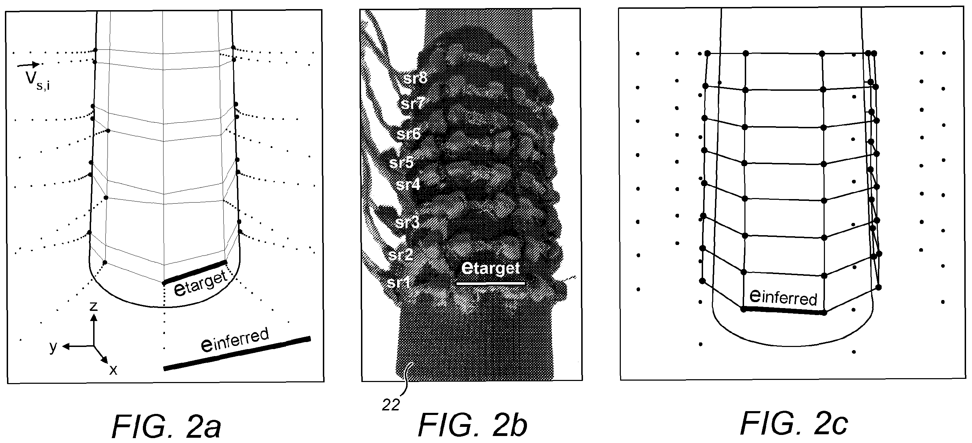

In one implementation with strain sensors, the strain sensors are located on the edge between nodes. The length of the edges between nodes relates to the elongation measured by strain sensors. For illustration, Figure 2(a) shows an elastic sleeve wrapping a cone, with the objective of reconstructing the shape of that cone based on the strain sensor readings. As the sensors are strain sensors, the model of (1) is a model which provides the expected reading of the strain sensor, based on the distance between nodes in the inferred shape (e.g. based on Hooke's law, or using some other approach such as lookup tables). This expected sensor reading is referred to as $e_{inferred}$. The actual readings of the strain sensors are referred to as $e_{target}$.

The loss function of (2) is defined as the sum of squared differences between the expected readings of the sensors, and the actual readings, for all the available sensors E:

$$L = \sum_{i=1}^{E} (e_{inferred,i} - e_{target,i})^2$$

The optimisation process of (3) starts from initial conditions of the inferred shape (i.e. initial shape), such as a cylinder with a diameter and length significantly larger than the expected target shape. A BFGS gradient descent algorithm (D.C. Liu et al., 1989, Mathematical Programming. 45, 503-528, Springer) was used to minimise the loss function L. The gradient descent computes a numerical approximation of the gradient by computing the partial derivative of the loss for a displacement of each of the nodes along each of X, Y and Z axes. Once the algorithm converges to an optima, the resulting mesh can be displayed and compared to the target. The inferred mesh after optimisation is indicated in Figure 2(a) as is the mesh as a result of the physical simulation.

SENSING TEXTILE

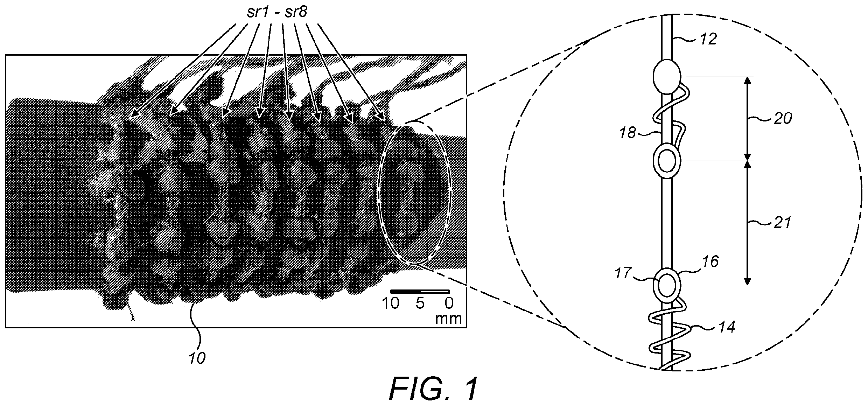

With reference to Figure 1, the sensing textile (10) developed was formed of eight sensing rings (sr1, sr2, sr3, sr4, sr5, sr6, sr7, sr8), each one comprising eight strain sensors (12) positioned one after the other. For practical implementation purposes there are no sensing elements between adjacent rings.

The sensing rings (sr1-sr8) were formed by attaching commercially available conductive rubber cord stretch sensors (12) (length 20 mm, diameter 2 mm, Adafruit, USA) (henceforth referred to as rubber sensors) onto a textile sleeve (Rymora Calf Compression Sleeves, Rymora Sports, UK).

The rubber sensors (12) were connected to enamelled copper wires (14) (diameter 0.19 mm) using a conductive epoxy (16) (CW2400, CircuitWorks, Chemtronics, Netherlands) at either end. Then, eight such sensors were attached onto an acrylic fettuccina yarn (18) (Yeoman Yarns, Leicester, UK) using sugru mouldable glue (Sugru Original formula, UK) to form the sensing rings (sr1-sr8).

The acrylic yarn was used as the carrier yarn and the copper wires were wrapped on it to prevent damage of the wires when the textile was stretched. The rubber sensors were positioned ≈5 mm apart from one another on the yarn.

The finalized structure had a rigid area (20) of 16.12±1.32 mm in between the active areas (21) of the rubber sensors (12). The next step was to attach these sensing rings onto the textile sleeve with a separation of 13.80±1.55 mm between each other. This was conducted in three steps, initially the rigid areas (20) of the sensing rings (sr1-sr8) were glued onto the surface of the textile sleeve using an adhesive (Evo-stick, UK). Then these rigid areas were sewn onto the sleeve and finally a layer of sugru was added on top of it to further secure the attachment. The textile sleeve had a slight conical topology, consequently, when the rings were attached onto the sleeve the distance between the active areas of the 1st and the 8th rubber sensor varied for each sensing ring. The 8th ring had a considerably smaller radius when compared to the rest. Therefore, the rigid area in between the 1st and 8th sensors was only 14.44 mm when compared to the rest (21.27±1.70 mm). The sensing textile (shown in Figure 1) was connected to an interface hardware circuit comprising four 16 channel analogue multiplexers (CD74HC4067) connected to an Arduino Uno board. An algorithm was written in MATLAB™ to record the measurements from the sensors.

CALIBRATION OF THE SMART TEXTILE

The sensing sleeve (10) was calibrated using four three-dimensional printed cylinders. The cylinders had diameters ranging from 58 mm to 64 mm. For the calibration, the resistance of each of the rubber sensors (12) was measured when the sleeve was at rest. Then the sleeve (10) was put on a cylinder and the resistance measurements were obtained after 10 min. The change in resistance ($\Delta R/R0$) was calculated using the resistance measurements, $\Delta R$ being the difference between the resistance at rest and when draped and R0 the resistance at rest. Thereafter the sensing sleeve (10) was taken off the cylinder and left 24 hours to recover. The sensors (12) were left to recover for this long since the preliminary response and recovery time experiments conducted had demonstrated that the rubber sensors took ≈3.1 h to completely recover once they were stretched to 50% strain and released. For these particular experiments, the rubber sensors were only stretched for <20% strain. The experiment was repeated up to three times for each of the cylinder diameters. The diameter of the cylinders along with the thickness of the sleeve were used to calculate the real circumference of the sensing rings. This was used to compute the elongation of each of the sensors. Thereafter, the polyfit function in MATLAB™ was utilised to create a first order polynomials using the resistance measurements and the elongation for each of the 64 sensors.

APPLICATION AND RESULTS

The sleeve draped two different three-dimensional printed cones (22). The elongation of the strain sensors was calculated utilising the resistance measurements capture from the strain sensors and the polynomial equations obtained. This elongation data was then added to the reconstruction algorithm and the cones were reconstructed as seen in Figure 2(c). The actual radius of the cones in the locations where the sensing rings were positioned were measured using a vernier caliper for comparison purposes.

The real radius of the cones and the reconstructed radius from the sensing rings and the reconstruction algorithm are given in Table 1. The reconstruction algorithm approximates the shape of the cone, as displayed in Figure 2(c). In cone 1, the average difference in between the radii computed by the reconstruction algorithm and the real measurements from the cone was 0.61±0.51 mm. This difference was mainly due to the measurements from the sensing rings 5 to 8. The rubber sensors in these rings did not elongate since the cone fitted loosely at the location of these rings. In the case of cone 2, the diameter of the cone was larger, therefore all the sensors were stretched ensuring a smaller average of difference of 0.44±0.22 mm.

Table 1: Real radii of the cones along with the radii computed by the reconstruction algorithm. sr=sensing ring.

|  | sr 1 (mm) | sr 2 (mm) | sr 3 (mm) | sr 4 (mm) | sr 5 (mm) | sr 6 (mm) | sr 7 (mm) | sr 8 (mm) |
|---|---|---|---|---|---|---|---|---|
| Real radius of cone 1 | 31.42 | 30.91 | 30.46 | 30.07 | 29.66 | 29.23 | 28.66 | 28.19 |
| Reconstructed radius of cone 1 | 30.85 | 31.01 | 30.38 | 30.04 | 30.13 | 30.35 | 29.93 | 29.49 |
| Real radius of cone 2 | 32.26 | 31.70 | 31.36 | 30.94 | 30.55 | 30.22 | 29.55 | 29.11 |
| Reconstructed radius of cone 2 | 31.38 | 31.25 | 30.77 | 30.42 | 30.30 | 30.36 | 30.06 | 29.35 |

EXAMPLE

**Fabrication and characterization of strain, pressure and temperature sensors**

The first step is to fabricate strain, pressure and temperature sensors embedded within the fibres of a textile yarn. The sensors have length <5.00 mm, width <0.75 mm and a height <0.01 mm to be integrated within textile yarns. The strain and pressure sensors are initially fabricated using solution-based fabrication methods where stretchable substrates, such as latex, are infused with conductive networks of graphene/silver nanowires. Ideally, the fabricated strain sensors have a gauge factor >8. In the case of pressure sensors, a sandwiched structure is utilized to fabricate a capacitive sensor with a sensitivity preferably >0.05 %/Pa. Their structure constitutes a latex layer positioned in the middle of two latex layers infused with a conductive network. For temperature sensors, standard microfabrication methods are used to fabricate Resistance Temperature Detectors (RTDs), where gold/chromium is patterned on a pre-stretched elastomer substrate. The RTDs preferably have a linear temperature sensitivity of >0.5 $\Omega$/°C and a resistance >1 k$\Omega$. A commercial/production friendly way of fabricating a strain/pressure/temperature sensor is by patterning a conductive ink (graphene composite TPU ink) onto an elastomer substrate (preferably Ecoflex/Thermoplastic Polyurethane/latex). This technique will ensure the fabrication of sensor with a stretchability >400 %. This method is implemented in the manufacturing of resistive strain sensors. For the pressure sensors, electrodes are printed on either side of a TPU/Ecoflex substrate to fabricate capacitive sensors. In the case of temperature sensors, RTDs are patterned by printing conductive electrodes onto the elastomer substrate. Several strain, pressure and temperature sensors are first patterned on an elastomer panel (35 mm by 15 mm). The panels are easy to fabricate and to handle. Thereafter the sensor panel is passivated using an epoxy such as SU-08.

**Equipment:** A class 1000 clean room is equipped with a thermal evaporator (BOC Edwards FL400), spin coater (Karl Suss RC 8) and UV mask aligner (Karl Suss) which is required for the fabrication of the temperature sensors. It also has a hot plate (IC20 chilling/heating dry bath Ecotherm) and stirrers needed for the synthesis of pressure and strain sensors. A high performance 3D printer (Optomec, Aerosol Jet HD) is used for the fabrication of the sensors. The printer is equipped with fine 20 $\mu$m print heads which assist in the fabrication of small sensor strips. A Stratasys Objet30 Prime 3D printer (PolyJet) has a precision of 36 $\mu$m.

**Integration of flexible sensors into textile yarns and textile sleeve fabrication**

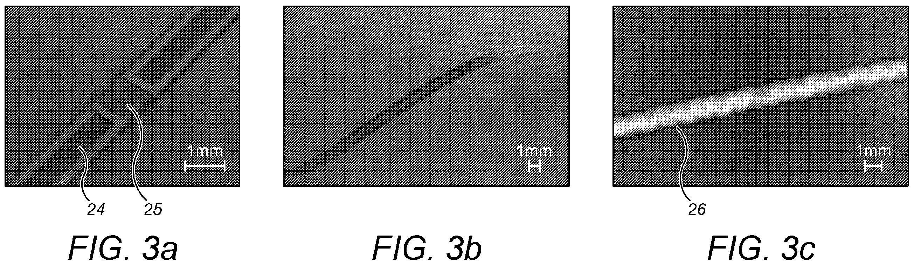

With reference to Figure 3, the integration of these sensors within the textile yarn requires the elastomer panels to be cut into sensors strips (24) of length <5.00 mm and width <0.75 mm. A laser cutter is used to cut the panels onto sensor strips. The strips are wired onto the interface hardware. The sensors are connected using low resistance enameled copper wires to ensure that there will be no overlapping of signals between the wires. These wires are connected to the elastomer structure using a silver conductive epoxy. The sensors are positioned on a carrier textile yarn (25) afterwards and encapsulated with a flexible PDMS adhesive. The encapsulation ensures the washability and biocompatibility of the fabricated device. The carrier yarn and the adhesive layer provide additional mechanical strength to the strips. The enameled copper wires are wrapped around the carrier yarn to prevent damage due to mechanical strain generated during the usage of the stretchable sleeve. The final step is the covering of the carrier yarns containing the flexible sensor strips with textile fibres (Figure 3(c)); commercially available yarn covering machines are used to achieve this. The textile cover (26) ensures that the sensors remained hidden and the feel of the textile is not altered. Suture braiders and knit braiding machines can be used to provide covers for flexible sensors. Once covered with textile filaments, the final yarn has a diameter ranging from 0.80 mm - 0.90 mm. Preliminary research has demonstrated the possibility of this integration technique. Eight strain/temperature/pressure sensors are positioned <5.00 mm apart within one textile yarn. Eight yarns are positioned one after the other separated <5.00 mm. This integration technique ensures that standard textile manufacturing processes such knitting, weaving and embroidery can be used for the fabrication of the sleeve. The sleeve is fabricated on a flatbed knitting machine. Once the yarns and the sleeve are fabricated, they are characterized against commercially available sensors. The reliability and the effects of hysteresis on them is evaluated by conducting cyclic tests on a Zwick/Roell machine.

**Equipment:** Laser cutters (techsoft lasercam) are used for the precise cutting of polymers and fabrication of the sensor strips. A prototype textile yarn covering machine is used to provide a prototype cover for the initial samples. For a more uniform yarn cover, knit braiders (RIUS MC braiding machine) and a suture braider are used. The fabrication of the sleeve is done on the flat bed knitting machine (Silver Reed SK840). The testing and characterisation of the yarns and sleeve is conducted using multimeters, parameter analysers, signal generator, hot plates, oscilloscope, etc.

**Design and assembly of the interface hardware and data processing software**

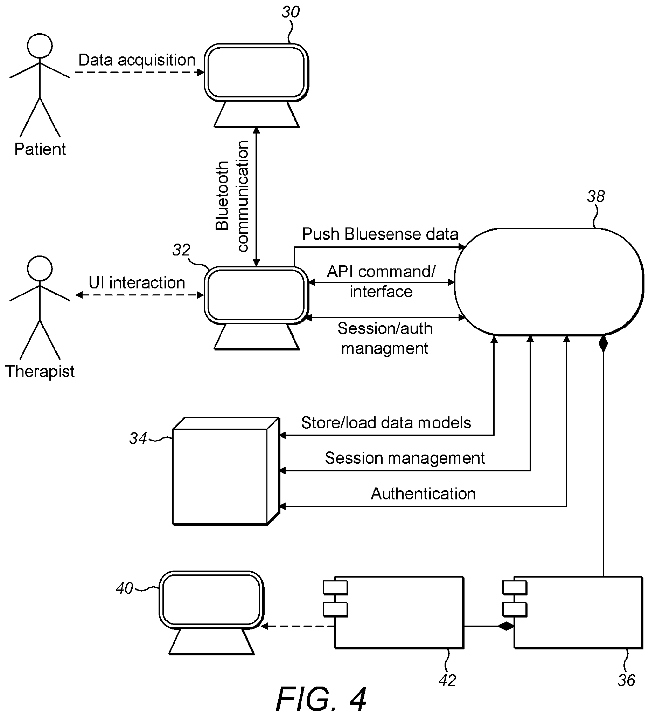

With reference to Figure 4, an interface hardware device (30) captures and conditions the sensor measurements. This device (30) transmits the data to a smartphone/personal computer (32) for further processing. The hardware comprises two CMOS multiplexers (32-channel) attached to a modified BlueSense device. Bluesense is a Bluetooth™ enabled microcomputer. It is battery powered and was chosen mainly due to its minute size (dimensions 30 mm by 30 mm).

Nevertheless, it has limited analogue input channels, therefore the sensors are connected through a multiplexer.

**Equipment:** BlueSense microcomputer, a PCB milling machine (LPKF) and equipment electronics (soldering stations, reflow ovens, etc.) to fabricate conventional interface and multiplexing boards for BlueSense.

**Design and development of a consumer friendly software system for data processing and real time visualization.**

A software developed for the acquisition and processing of data fetched from the textile body shape sensor devices, is able to perform an accurate real time 3D reconstruction. Compatibility and portability are mandatory requirements to support mobile and portable personal devices such as laptops, smartphones, or tablets with Android operative systems.

To satisfy high demanding time and space computational requirements, the software system architecture is a distributed Service Oriented Architecture (SOA), as shown in Figure 4. As such, it includes a persistence layer for data storage (34) and a web service business logic component (36). This approach facilitates a scalable and modular approach to minimize the impact of future requirements or change requests.

The textile body shape sensor is connected to the BlueSense microcomputer (30) to read and transmit the data. The BlueSense equipment establishes communication with a portable Android device (32). The latter have multiple responsibilities, among which are offering control and data presentation interface to the therapist through a graphical Human-Computer Interface (HCI). This interface exposes simple and advanced modes to facilitate users with small or no experience with computer aided design (CAD) software. In addition, the Android device delegates computing demanding and data persistence tasks to a Microsoft IIS web service (38) which runs the core business logic. Among the responsibilities of the server is the authentication and session management, as well as the processing of data models in available formats, such as STL, or XYZ, that will be compatible with commercial CAD software (e.g. Solidworks), through a SOA web service API. Additionally, the web server application is responsible for computing tasks including the reconstruction algorithms to overcome the computing limitations of portable devices. Such extensible architectural approach also enables the easy implementation of 3D format converters as internal or third-party plugins. To better support multiple Computer Aided Manufacturing (CAM) devices (40), such as Computer Numerical Control (CNC) machines, an adapter interface component (42) has been designed and linked to the core business logic (36).

It should be understood that various changes and modifications to the presently preferred embodiments described herein will be apparent to those skilled in the art. Such changes and modifications can be made without departing from the scope of the present invention and without diminishing its attendant advantages.

**Claims**

1. A method for providing a customized orthotic product, comprising:

   providing an article, comprising a stretchable material, and a plurality of strain sensors positioned in contact with the material such that stretching of the material is detectable by the sensors;
   placing the article onto a body part so that the material is stretched, at least in part;
   providing a computing device operatively coupled to the article and configured to receive output data from the sensors and to process the data to determine the three-dimensional shape of the body part;
   applying force, deformation pressure, rotation and/or flexion at one or more target regions of the body part to hold, manipulate and/or correct the body part;
   reading output from the sensors, and determining the three-dimensional shape of the body part based on the output from the sensors;
   using the determined three-dimensional shape of the body part to design and/or fabricate a customized orthotic product.

2. A method according to claim 1, wherein the force, deformation pressure, rotation and/or flexion to the target region(s) is applied by a practitioner's hands.

3. A method according to claim 1 or claim 2, wherein sensors at the one or more target regions are configured to detect at least one of pressure, compression, rotation, flexion and temperature.

4. A method according to claim 1 or claim 2, further comprising displaying the three-dimensional shape of the body part on a display.

5. A method according to any one of the preceding claims, wherein the three-dimensional image of the shape of the body part is used to design a customized orthotic product by (1) fabricating the body part in three-dimensional form and designing the orthotic product using the fabricated body part, or (2) designing the orthotic product using the three-dimensional image of the body part, and fabricating the orthotic product in three-dimensional form from the designed image of the orthotic product.

6. A method according to any one of the preceding claims, wherein the three-dimensional shape of the body part is determined from sensor data using an algorithm/model that links inferred shape geometry to expected sensor reading; a loss function (L), measuring the degree of agreement between the sensor readings and the readings that would be expected based on the geometry of the inferred shape; and an optimisation function, iteratively modifying the inferred shape in order to minimise the value of the loss function.

7. A method according to any one of the preceding claims, wherein the sensors are (a) integrated or embedded within the material and/or positioned on the material surface; and/or (b) provided in channels or the like formed in the material.

8. A method according to any one of the preceding claims, wherein the sensors are laminated on an elastomeric substrate, optionally wherein the substrate is in the form of a strip.

9. A method according to any one of the preceding claims, wherein the material is a textile and the sensors are positioned on, or integrated or embedded within, at least one yarn or fibre of the textile; optionally wherein the article is knitted or woven.

10. A method according to any one of the preceding claims, wherein the sensors (a) are positioned less than about 5 mm apart, such as about 4 mm, about 3 mm, about 2 mm or about 1 mm apart; and/or (b) have a height or thickness of less than about 0.02 mm, less than about 0.015 mm or less than about 0.01 mm, such as 9 $\mu$m, 8 $\mu$m, 7 $\mu$m, 6 $\mu$m or 5 $\mu$m; and/or (c) have a width of less than about 1.5 mm, less than about 1.0 mm, less than about 0.85 mm, less than about 0.80 mm, or less than about 0.75 mm, such as 0.6 mm, 0.5 mm, 0.4 mm, 0.3 mm, 0.2 mm or 0.1 mm.

11. A method according to any one of the preceding claims, wherein an individual yarn including sensors is encapsulated within a flexible cover, optionally a moisture or water-resistant flexible cover, optionally wherein the diameter of the individual yarn with cover is about 2.0 mm or less, preferably about 0.7 mm to about 1.0 mm, such as about 0.75 mm to about 0.95 mm or about 0.8 mm to about 0.9 mm.

12. A method according to any one of the preceding claims, wherein the sensors are coated to provide a washable article; and/or wherein the article is in the form of a sleeve adapted to snugly fit over a part of the object, for example a body part, such as a limb of a patient.

13. A method according to any one of the preceding claims, further comprising one or more sensors configured to detect at least one of pressure, compression, force, temperature, volume, blood oxygenation, pH, chemicals, skin surface moisture, flexion and rotation.

**Patentansprüche**

1. Verfahren zum Bereitstellen eines individuellen orthopädischen Produkts, umfassend:

Bereitstellen eines Artikels, der ein dehnbares Material und mehrere Dehnungssensoren umfasst, die in Kontakt mit dem Material angeordnet sind, so dass die Dehnung des Materials von den Sensoren erfasst werden kann;
Anlegen des Artikels an ein Körperteil, so dass das Material zumindest teilweise gedehnt wird;
Bereitstellen einer Computervorrichtung, die betriebsmäßig mit dem Artikel gekoppelt und konfiguriert ist, Ausgangsdaten von den Sensoren zu empfangen und die Daten zu verarbeiten, um die dreidimensionale Form des Körperteils zu bestimmen;
Aufbringen von Kraft, Verformungsdruck, Drehung und/oder Biegung auf einen oder mehrere Zielbereiche des Körperteils, um den Körperteil zu halten, zu manipulieren und/oder zu korrigieren;
Lesen der Ausgabe von den Sensoren und Bestimmen der dreidimensionalen Form des Körperteils auf der Grundlage der Ausgabe von den Sensoren;
Verwendung der bestimmten dreidimensionalen Form des Körperteils zum Entwerfen und/oder Herstellen eines individuelles orthopädischen Produkts.

**2.** Verfahren nach Anspruch 1, wobei die Kraft, der Verformungsdruck, die Drehung und/oder die Biegung auf den/die Zielbereich(e) durch die Hände eines Arztes ausgeübt wird.

**3.** Verfahren nach Anspruch 1 oder Anspruch 2, worin die Sensoren an der einen oder den mehreren Zielregionen ausgestaltet sind, mindestens einen der Parameter Druck, Kompression, Drehung, Biegung und Temperatur erfassen.

**4.** Verfahren nach Anspruch 1 oder Anspruch 2, welches ferner das Anzeigen der dreidimensionalen Form des Körperteils auf einem Display umfasst.

**5.** Verfahren nach einem der vorstehenden Ansprüche, wobei das dreidimensionale Bild der Form des Körperteils verwendet wird, um ein individuelles orthopädisches Produkt zu entwerfen, indem (1) das Körperteil in dreidimensionaler Form hergestellt wird und das orthopädische Produkt unter Verwendung des hergestellten Körperteils entworfen wird, oder (2) das orthopädische Produkt unter Verwendung des dreidimensionalen Bildes des Körperteils entworfen wird und das orthopädische Produkt in dreidimensionaler Form aus dem entworfenen Bild des orthopädischen Produkts hergestellt wird.

**6.** Verfahren nach einem der vorstehenden Ansprüche, wobei die dreidimensionale Form des Körperteils aus Sensordaten unter Verwendung eines Algorithmus/Modells bestimmt wird, der/das die abgeleitete Formgeometrie mit den erwarteten Sensormesswerten verknüpft; eine Lass-Funktion (L), die den Grad der Übereinstimmung zwischen den Sensormesswerten und den Messwerten misst, die auf der Grundlage der Geometrie der abgeleiteten Form zu erwarten wären; und eine Optimierungsfunktion, die die abgeleitete Form iterativ modifiziert, um den Wert der Lass-Funktion zu minimieren.

**7.** Verfahren nach einem der vorstehenden Ansprüche, worin die Sensoren (a) in das Material integriert oder eingebettet und/oder auf der Materialoberfläche angeordnet sind; und/oder (b) in Kanälen oder dergleichen vorgesehen sind, die in dem Material ausgebildet sind.

**8.** Verfahren nach einem der vorstehenden Ansprüche, worin die Sensoren auf ein elastomeres Substrat laminiert sind, worin das Substrat wahlweise die Form eines Streifens aufweist.

**9.** Verfahren nach einem der vorstehenden Ansprüche, worin das Material ein Textil ist und die Sensoren auf mindestens einem Garn oder einer Faser des Textils angeordnet oder darin integriert oder eingebettet sind; wahlweise worin der Artikel gestrickt oder gewebt ist.

**10.** Verfahren nach einem der vorstehenden Ansprüche, worin die Sensoren (a) weniger als etwa 5 mm voneinander entfernt angeordnet sind, wie etwa 4 mm, etwa 3 mm, etwa 2 mm oder etwa 1 mm; und/oder (b) eine Höhe oder Dicke von weniger als etwa 0,02 mm, weniger als etwa 0,015 mm oder weniger als etwa 0,01 mm, wie etwa 9 $\mu$m, 8 $\mu$m, 7 $\mu$m, 6 $\mu$m oder 5 $\mu$m; und/oder (c) eine Breite von weniger als etwa 1,5 mm, weniger als etwa 1,0 mm, weniger als etwa 0,85 mm, weniger als etwa 0,80 mm oder weniger als etwa 0,75 mm, wie etwa 0,6 mm, 0,5 mm, 0,4 mm, 0,3 mm, 0,2 mm oder 0,1 mm aufweisen.

**11.** Verfahren nach einem der vorstehenden Ansprüche, worin ein einzelner Faden, der Sensoren enthält, in eine flexible Umhüllung eingekapselt ist, wahlweise eine feuchtigkeits- oder wasserbeständige flexible Umhüllung, wahlweise worin der Durchmesser des einzelnen Fadens mit der Umhüllung etwa 2,0 mm oder weniger beträgt, vorzugsweise etwa 0,7 mm bis etwa 1,0 mm, wie etwa 0,75 mm bis etwa 0,95 mm oder etwa 0,8 mm bis etwa 0,9 mm.

**12.** Verfahren nach einem der vorstehenden Ansprüche, worin die Sensoren beschichtet sind, um einen waschbaren Artikel bereitzustellen; und/oder worin der Artikel die Form einer Manschette aufweist, die angepasst ist, eng über einen Teil des Objekts zu passen, beispielsweise einem Körperteil, wie einer Gliedmaße eines Patienten.

**13.** Verfahren nach einem der vorstehenden Ansprüche, welches ferner einen oder mehrere Sensoren umfasst, die konfiguriert sind, mindestens einen der folgenden Parameter zu erfassen: Druck, Kompression, Kraft, Temperatur, Volumen, Blutsauerstoffgehalt, pH-Wert, Chemikalien, Feuchtigkeit der Hautoberfläche, Biegung und Drehung.

**EP 4 027 877 B1**

**Revendications**

1. Procédé de fourniture d'un produit orthétique personnalisé, comprenant :

   la fourniture d'un article, comprenant un matériau extensible, et d'une pluralité de capteurs de contrainte positionnés en contact avec le matériau de sorte que l'étirement du matériau soit détectable par les capteurs ;
   le placement de l'article sur une partie du corps de sorte que le matériau soit étiré, au moins en partie ;
   la fourniture d'un dispositif informatique couplé de manière opérationnelle à l'article et configuré pour recevoir des données de sortie des capteurs et pour traiter les données pour déterminer la forme tridimensionnelle de la partie du corps ;
   l'application d'une force, pression de déformation, rotation et/ou flexion au niveau d'une ou plusieurs régions cibles de la partie du corps pour maintenir, manipuler et/ou corriger la partie du corps ;
   la lecture de la sortie des capteurs, et la détermination de la forme tridimensionnelle de la partie du corps sur la base de la sortie des capteurs ;
   l'utilisation de la forme tridimensionnelle déterminée de la partie du corps pour concevoir et/ou fabriquer un produit orthétique personnalisé.

2. Procédé selon la revendication 1, dans lequel la force, pression de déformation, rotation et/ou flexion sur la ou les régions cibles est appliquée par les mains d'un praticien.

3. Procédé selon la revendication 1 ou revendication 2, dans lequel des capteurs au niveau des une ou plusieurs régions cibles sont configurés pour détecter au moins l'une d'une pression, compression, rotation, flexion et température.

4. Procédé selon la revendication 1 ou revendication 2, comprenant en outre l'affichage de la forme tridimensionnelle de la partie du corps sur un écran.

5. Procédé selon l'une quelconque des revendications précédentes, dans lequel l'image tridimensionnelle de la forme de la partie du corps est utilisée pour concevoir un produit orthétique personnalisé en (1) fabriquant la partie du corps sous forme tridimensionnelle et concevant le produit orthétique à l'aide de la partie du corps fabriquée, ou (2) concevant le produit orthétique à l'aide de l'image tridimensionnelle de la partie du corps, et en fabriquant le produit orthétique sous forme tridimensionnelle à partir de l'image conçue du produit orthétique.

6. Procédé selon l'une quelconque des revendications précédentes, dans lequel la forme tridimensionnelle de la partie du corps est déterminée à partir de données de capteur à l'aide d'un algorithme/modèle qui lie la géométrie de forme déduite à la lecture de capteur attendue ; une fonction de perte (L), mesurant le degré d'accord entre les lectures de capteur et les lectures qui seraient attendues sur la base de la géométrie de la forme déduite ; et une fonction d'optimisation, modifiant de manière itérative la forme déduite afin de minimiser la valeur de la fonction de perte.

7. Procédé selon l'une quelconque des revendications précédentes, dans lequel les capteurs sont (a) intégrés ou encastrés dans le matériau et/ou positionnés sur la surface du matériau ; et/ou (b) prévus dans des canaux ou similaires formés dans le matériau.

8. Procédé selon l'une quelconque des revendications précédentes, dans lequel les capteurs sont stratifiés sur un substrat élastomère, facultativement dans lequel le substrat se présente sous la forme d'une bande.

9. Procédé selon l'une quelconque des revendications précédentes, dans lequel le matériau est un textile et les capteurs sont positionnés sur, ou intégrés ou incorporés dans, au moins un fil ou une fibre du textile ; facultativement dans lequel l'article est tricoté ou tissé.

10. Procédé selon l'une quelconque des revendications précédentes, dans lequel les capteurs (a) sont positionnés à une distance inférieure à environ 5 mm, telle qu'environ 4 mm, environ 3 mm, environ 2 mm ou environ 1 mm l'un de l'autre ; et/ou (b) ont une hauteur ou épaisseur inférieure à environ 0,02 mm, inférieure à environ 0,015 mm ou inférieure à environ 0,01 mm, telle que 9 $\mu$m, 8 $\mu$m, 7 $\mu$m, 6 $\mu$m ou 5 $\mu$m ; et/ou (c) ont une largeur inférieure à environ 1,5 mm, inférieure à environ 1,0 mm, inférieure à environ 0,85 mm, inférieure à environ 0,80 mm, ou inférieure à environ 0,75 mm, telle que 0,6 mm, 0,5 mm, 0,4 mm, 0,3 mm, 0,2 mm ou 0,1 mm.

11. Procédé selon l'une quelconque des revendications précédentes, dans lequel un fil individuel incluant des capteurs est encapsulé dans un revêtement flexible, facultativement un revêtement flexible résistant à l'humidité ou à l'eau,

13

facultativement dans lequel le diamètre du fil individuel avec revêtement est d'environ 2,0 mm ou moins, de préférence d'environ 0,7 mm à environ 1,0 mm, tel que d'environ 0,75 mm à environ 0,95 mm ou d'environ 0,8 mm à environ 0,9 mm.

12. Procédé selon l'une quelconque des revendications précédentes, dans lequel les capteurs sont revêtus pour fournir un article lavable ; et/ou dans lequel l'article se présente sous la forme d'un manchon adapté pour s'ajuster sur une partie de l'objet, par exemple une partie du corps, telle qu'un membre d'un patient.

13. Procédé selon l'une quelconque des revendications précédentes, comprenant en outre un ou plusieurs capteurs configurés pour détecter au moins l'un des pression, compression, force, température, volume, oxygénation du sang, pH, produits chimiques, humidité de la surface de la peau, flexion et rotation.

*FIG. 1*

FIG. 2a

FIG. 2b

FIG. 2c

*FIG. 3c*

*FIG. 3b*

*FIG. 3a*

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2014276235 A

- WO 2012168836 A

**Non-patent literature cited in the description**

- **D.C. LIU et al.** Mathematical Programming. Springer, 1989, vol. 45, 503-528